# EUROPEAN PATENT SPECIFICATION

(11) **EP 2 410 030 B1**
(45) Date of publication and mention of the grant of the patent: **12.10.2016**
(21) Application number: 10753141.0
(22) Date of filing: 18.03.2010
(51) Int. Cl.: C09F 1/02, C07C 61/08, C07C 51/42, C07D 307/77, C07D 307/89

(54) **PREPARATION METHOD OF PIMARIC ACID TYPE RESIN ACID**
VERFAHREN ZUR HERSTELLUNG EINES KUNSTHARZES AUS PIMARINSÄURE
MÉTHODE DE SYNTHÈSE D'UN ACIDE RÉSINIQUE DE TYPE ACIDE PIMARIQUE

(30) Priority: 20.03.2009 CN 200910030374
(43) Date of publication of application: 25.01.2012
(73) Proprietor: Institute Of Chemical Industry Of Forest Products, Chinese Academy Of Forestry, Jiangsu 210042 (CN)
(72) Inventor: ZHAO, Zhendong, Nanjing Jiangsu 210042 (CN); CHEN, Yuxiang, Nanjing Jiangsu 210042 (CN); BI, Liangwu, Nanjing Jiangsu 210042 (CN); GU, Yan, Nanjing Jiangsu 210042 (CN); LI, Dongmei, Nanjing Jiangsu 210042 (CN); WANG, Jing, Nanjing Jiangsu 210042 (CN); LU, Yanju, Nanjing Jiangsu 210042 (CN)
(74) Representative: dompatent von Kreisler Selting Werner - Partnerschaft von Patent- und Rechtsanwälten mbB
(86) International application number: PCT/CN2010/071134
(87) International publication number: WO 2010/105574

(56) References cited:
- CN-A- 101 020 630
- CN-A- 101 302 151
- CN-A- 101 508 871
- US-A- 3 658 891
- US-A- 3 658 891
- DATABASE CA [Online] CHEMICAL ABSTRACTS SERVICE, COLUMBUS, OHIO, US; 2009, WANG, HONG-XIAO ET AL: "Synthesis of maleopimaric acid under microwave irradiation", XP002686985, retrieved from STN Database accession no. 2009:372892 & WANG, HONG-XIAO ET AL: "Synthesis of maleopimaric acid under microwave irradiation", HUAXUE SHIJI , 31(3), 177-179, 202 CODEN: HUSHDR; ISSN: 0258-3283, March 2009 (2009-03),
- HONGHUA WANG ET AL: "Synthesis of biobased epoxy and curing agents using rosin and the study of cure reactions", GREEN CHEMISTRY, vol. 10, no. 11, 2008, pages 1190-1196, XP002686986, ROYAL SOCIETY OF CHEMISTRY, CAMBRIDGE, ISSN: 1463-9262
- GEORGE, GONIS ET AL.: 'Preparation of Maleopimaric Acid' IND.ENG.CHEM.PROD.RES.DEVELOP. vol. 12, no. 4, 1973, pages 326 - 327, XP055098380
- G.C.HARRIS ET AL: "Resin acids. III. The isolation of dextopimaric acid and a new pimaric-type acid, isodextopimaric acid", JOURNAL OF THE AMERICAN CHEMICAL SOCIETY., vol. 70, 1948, pages 2079-2081, USAMERICAN CHEMICAL SOCIETY, WASHINGTON, DC. ISSN: 0002-7863

## Description

### Field of the Invention

The present invention relates to a process for preparing resin acid, in particular pimaric type resin acid, from pine oleoresin or rosin.

### Background of the Invention

Pine oleoresin is mainly comprised of a series of unsaturated diterpene carboxylic acids having phenanthrene ring structure, except turpentine, a few neutral compounds, and a few fatty acids. These diterpene carboxylic acids have the same molecular formula C₂₀H₃₀O₂, and are generally known as resin acids. The resin acids mainly include abietic acid, palustric acid, levopimaric acid, neoabietic acid, dehydrogenated abietic acid, pimaric acid, isopimaric acid, and sandaracopimaric acid, etc. These resin acids can be divided into two categories: abietic type resin acids and pimaric type resin acids by chemical structure. The molecule of abietic type resin acid has two conjugated double bonds and one isopropyl. Abietic acid, palustric acid, and levopimaric acid, as well as dehydrogenated abietic acid all belong to this category.

The molecule of pimaric type resin acid has a methyl and a vinyl at the position of C₁₃, and has two separate double bonds. This category mainly includes pimaric acid resin acids which are commonly seen in pine oleoresin and rosin, such as pimaric acid, isopimaric acid, and sandaracopimaric acid, and pimaric type resin acids which are rarely seen in the natural world, such as 8,15-isopimaric acid, Δ8(9)-pimaric acid, 7,15-pimaradiene-18-acid, etc..

Some components of pimaric type resin acids have biological activities against cancer, viruses, phlogosis, bacteria, and parasites, etc., and have potential therapeutic actions against diseases such as hypertension, tonic cystitis, allergic bronchitis, chemical central nervous system disorders. They can also be transformed into derivatives by utilizing the active groups (e.g., carboxyl, exocyclic vinyl) in the molecular structure and then be utilized again.

There are few reports on the research of the method for separation and preparation of some components in pimaric type resin acids:
(a) Alkali metal salt method [PALKIN S, HARRIS T H. The resin acids of American Turpentine Gum: The preparation of the pimaric acids from Pinus Palustris [J]. J Am Chem Soc, 1933, 55(9): 3677-3684; Sandermann W. Chemistry and Technology of Natural Resin, Turpentine, and Wood Pulp Oil Slick [M]. Beijing: China Forestry Publishing House, 1982:56]. In this method, pine oleoresin is treated by vacuum filtration first to obtain fresh resin acids, then the resin acids are extracted with 80% ethanol, and the residue of extraction is recrystallized with 95% ethanol to obtain the mixture of levopimaric acid and pimaric acid; then, the mixture is transformed into sodium salt which can be recrystallized to obtain pimaric acid. However, the yield of pimaric acid is only 3.3%. In this method, the temperature of extraction and crystallization must be controlled strictly, and pine oleoresin chosen as the raw material must ensure the optical activity of sodium salt is higher than -160°. The method reported by Vesterberg is: the crystallinic resin acid obtained from galipia officinalis resin or French rosin is first produced into pimaric ammonium salt, which is then transformed into pimaric sodium salt; finally, the pimaric sodium salt is recrystallized in 2% NaOH. However, the yield of pimaric type resin acid is only 1.5% of galipia officinalis resin. In this method, pimaric acid experiences many transformation procedures; therefore, the loss is severe and the yield is low. This method is not suitable for large-scale preparation.
(b) Direct ammonium salt precipitation method [LOEBLICH V M, LAWRENCE R V A new method for isolating isodextropimaric acid from pine oleoresin and rosin [J]. J Org Chem, 1958, 23(1): 25-26]. In this method, isopimaric ammonium salt is first precipitated directly from n-heptane solution of rosin with piperidine; then the ammonium salt is purified by fractional crystallization with 95% ethanol as the solvent; finally, the ammonium salt is reduced to isopimaric acid. Although piperidine is selective to isopimaric acid, this method has a shortcoming, namely, the crystallization rate of the produced ammonium salt is very slow, and the yield is low. Therefore, that method can only be used for research in small quantities. Recently, in Chinese Patent Application No. CN101302151A filed by Zhao Zhendong, et al, a method for preparing isopimaric acid is disclosed. In this method, thermally isomerized rosin is dissolved in acetone, and treated with isobutanolamine to form a crude product of isopimaric ammonium salt; then, the isopimaric ammonium salt is purified by multi-recrystallization method, and then treated with acidification and ionization via hydrochloric acid, and finally refined and purified to obtain purified isopimaric acid. Although this method has advantages such as low cost and high yield, it is not suitable for preparing mixed pimaric type resin acids.
(c) Maleated derivatization method [HARRIS G C, SANDERSON T F. Rosin acids (III) The isolation of dextropimaric acid, a new pimaric-type acid, isodextropimaric acid [J]. J Am Chem Soc, 1948, 70(1): 2079-2085; ALDRICH P H. Process for separation of rosin adducts from mixtures with rosin: US, 3562243[P], 1971]. In this method, ammonium salt is produced from the reaction between cyclohexylamine and resin acids; then, the ammonium salt is treated by crystallization, separation, and acidification to obtain a purified resin acid mixture; then, the resin acid mixture is catalyzed by saturated hydrogen chloride to react with maleic anhydride in benzene solvent under boiling condition for 24h; after the benzene solvent is distilled, the residue is dissolved in concentrated alkaline solution; then, the pH of the solution is adjusted to 6.2, to make the unreacted resin acid crystallize. Then, the unreacted resin acid reacts with butanolamine (2-amido-2-methyl-1-propanol) in acetone solution to obtain the crystalline of isopimaric ammonium salt; then, the isopimaric ammonium salt is recrystallized with methyl acetate, and treated by acidification, ionization, and purification to obtain isopimaric acid. Aldrich et al studied several processes for separating maleated adducts, including crystallization with glacial acetic acid, dissolution and separation of carbon tetrachloride adduct, and dissolution by means of solvent polarity difference, etc. The target product of the above method is isopimaric acid. The shortcoming of this method is long reaction time; in addition, it is difficult to completely separate maleopimaric acid and unreacted resin acids, including pimaric type resin acids, dehydro abietic acid and a small amount of abietic acid-type resin acid that is not thoroughly reacted; as a result, the product may contain maleopimaric acid. Moreover, wide use of benzene solvent and hydrogen chloride gas may cause serious environmental pollution and high requirement for the equipment.
(d) Benzoquinone derivatization method [Sandermann W. Chemistry and Technology of Natural Resin, Turpentine, and Wood Pulp Oil Slick [M]. Beijing: China Forestry Publishing House, 1982: 56]. In this method, the resin acids from pine resin is transformed into benzoquinone adducts; the benzoquinone adducts are filtered, and the crystalline product obtained from the mother solution is crystalized in acetone, and then recrystallized in glacial acetic acid and methanol to obtain pimaric type resin acids. A drawback of this method is: the application approaches of the benzoquinone adducts is very limited, and therefore may cause loss of raw material and increased cost. This method needs further improvements in terms of economic efficiency.
(e) Rectification method [HARRIS G C, SANDERSON T F. Rosin acids (III) The isolation of dextropimaric acid, a new pimaric-type acid, isodextropimaric acid [J]. J Am Chem Soc, 1948, 70(1): 2079-2085]. In this method, fatwood rosin or gum rosin is rectified in a tower having 10 tower plates at 13.3Pa pressure; the fraction with boiling point between 136∼200°C is taken to produce ether solution of sodium salt; the sodium salt is treated by acidification, the resin acids are dissolved in ether; then, ether is removed to obtain concentrated pimaric acids and isopimaric acids. A drawback of the method is the high requirements for fractionation condition, which is adverse to operation; in addition, the yield of isopimaric acids and pimaric acids is low.

At present, there are few research and development on separation and purification of pimaric type resin acids in the world, and nearly no report on developing and utilizing pimaric type resin acids as a product. Up to now, there is no commercial pimaric type resin acid product or rosin product rich in pimaric type resin acid yet. Exploring high-efficiency, high-yield, and high economic feasibility methods for preparation of pimaric type resin acids can facilitate and speed up the research and development of application of pimaric type resin acid products, especially in medicine, biology, material and other fields.

### Summary of the Invention

In view of the drawbacks in the prior art, such as low yields, severe environmental pollution, and high costs, etc., the present invention provides a process for preparing pimaric type resin acids, which has advantages such as high yield, high content, low costs, and less environmental pollution, etc.

The present invention employs the following technical scheme:
A process for preparing pimaric type resin acids, comprising the following steps:
   Step 1: load refined resin acids, pine resin or rosin, and maleic anhydride at 1:0.3∼1.5 mass ratio into a reaction flask, dissolve them in C₁∼C₁₀ lower fatty acid solvent, the mass ratio between C₁∼C₁₀ lower fatty acid solvent and refined resin acids being 0.05~30:1, and then let them have additive reaction under microwave-assisted heating or direct heating; after the reaction, perform the step of cooling, crystallization, filtration and cleaning; said C₁∼C₁₀ lower fatty acid solvent is glacial acetic acid, propionic acid, or butyric acid. During microwave-assisted heating, the microwave power is 100W∼50kW, the reaction time under microwave heating is 5min∼300min, and the reactor is a special refractory reactor that doesn't absorb microwave and is equipped with a reflux condenser.
   Step 2: combine the filtrate collected in step 1, and then perform reduced pressure distillation to remove the solvent to obtain the crude product of pimaric type resin acids; then, dissolve the crude product of pimaric type resin acids in sodium hydroxide solution to produce saline solution of pimaric type resin acids; then, adjust the pH to 6∼14 with mineral acid or organic during agitation; treat the obtained precipitate with direct purification or purification after acidification, to obtain the final product of pimaric type resin acids. The concentration of the sodium hydroxide solution is 0.5wt%∼40wt%. Said mineral acid is hydrochloric acid or sulfuric acid, the organic acid is formic acid or acetic acid, and the concentration of the acid is 0.5wt%∼100wt%. The purification process is to dissolve the precipitate in ether with a mass of 0.5~20 times than the precipitate , wash with water till the ether layer become neutral, and then dry with anhydrous magnesium sulfate; then, evaporate ether at 1 atm, and finally dry in vacuum to obtain the product of pimaric type resin acids.

The refined resin acids are purified resin acids which are obtained by removing the neutral substances in pine oleoresin or rosin through recrystallization in organic solvent, treating with sodium salt, treating with ammonium salt, or washing with organic solvent, etc. Specifically, dissolve 1 pbw (part by weight) of pine oleoresin or rosin in 2.5 pbw petroleum ether with 60°C∼90°C boiling range while heating, remove insoluble solid impurities by filtering, remove water from the filtrate with a separatory funnel, slowly add cyclohexylamine solution in drops while agitating to produce a large quantity of white precipitate of ammonium salt of resin acid, the cyclohexylamine solution is prepared by dissolving cyclohexylamine in quantity equal to the molar quantities of the resin acids contained in the pine oleoresin or rosin into 0.4 pbw petroleum ether. Agitate for 1h at 40°C, and then cool down to room temperature, and then, further cool down in ice water bath, and then perform vacuum filtration, wash the precipitate with 0.2 pbw petroleum ether for 3 times, and dry in vacuum at 40°C, then grind the white resin acid ammonium salt into powder, add 0.7 pbw ether, add 2mol/L hydrochloric acid solution during agitation at room temperature, till the white resin acid ammonium salt powder disappears completely, and then agitate further for 30min.,transfer the mixture into a separatory funnel to remove the water layer, and then wash with distilled water, till the pH of the water phase is 6; then, dry with anhydrous sodium sulfate after separation, and then dry in vacuum at 40°C, to obtain refined resin acids.

The pine oleoresin used as raw material in the present invention can be pine oleoresin that contains pimaric type resin acids, such as *Pinus massoniana* pine oleoresin, *Pinus elliottii* pine oleoresin, *Pinus Caribaea* pine oleoresin, *Pinus khasya* pine oleoresin, etc., wherein pine oleoresin rich in pimaric type resin acids, such as *Pinus elliottii* pine oleoresin, is the best choice; the rosin used as raw material in the present invention can be rosin that contains pimaric type resin acids, such as *Pinus massoniana* rosin, *Pinus elliottii* rosin, *Pinus Caribaea* rosin, *Pinus khasya* rosin, etc., wherein rosin rich in pimaric type resin acids, such as *Pinus elliottii* rosin, is the best choice; the rosin may be gum rosin, tall oil rosin, and wood rosin; the resin acids used as raw material in the present invention are preferably refined resin acids obtained from pine oleoresin or rosin through pre-treatment including salinization, acidification or recrystallization, etc.

The chemical structural formula of abietic acid type resin acids and the principle of maleation reaction are shown as follows:

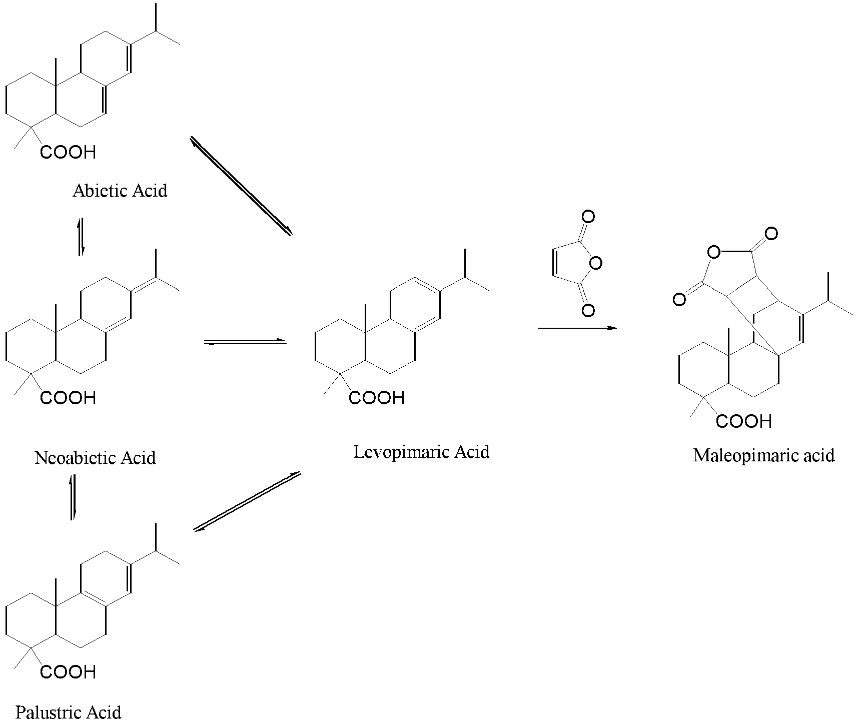

Beneficial effects:
1. The present invention adopts microwave-assisted heating in maleation reaction, during which the abietic acid type resin acids in the raw material are transformed into maleopimaric acid. The principle are as follows: under the action of a high-frequency electromagnetic field, polar molecules tend to orient according to the polarity of the electromagnetic field from the original random distribution state, and the orientation varies according to the frequency of the alternating electromagnetic field; in this process, molecules move and rub against each other to produce heat; at the same time, the polar molecules that have absorbed energy transfer energy to other molecules when they collide with other molecules, causing increased medium temperature. Since the reaction duration of additive reaction under microwave assistance is short and the transformation ratio of abietic acid type resin acids is as high as 97% or above, the content of abietic acid-type resin acids in the pimaric type resin acid product is very low; therefore, the content and yield of the target product (pimaric type resin acids) are both increased, wherein, the content can be 93.5% or above, and the yield can be 64.4% or above.
2. The present invention use C₁∼C₁₀ lower fatty acid (e.g., glacial acetic acid) as the reaction medium, which can absorb microwave nicely; in addition, the acidic property of organic acid has catalytic effect to the maleation reaction, and therefore shortens the reaction time effectively (from original 4h or longer to 1h or shorter duration), facilitates the transformation of abietic acid type resin acids into maleopimaric acid, and further increases the yield of the target product (pimaric type resin acids).
3. The present invention use organic acid (e.g., glacial acetic acid) as the solvent for recrystallization, so most of the produced maleopimaric acid can be removed effectively, thus, the adverse effect of a large quantity of maleopimaric acid entrained in the precipitation in the following pH adjustment procedure is reduced effectively, and the quality of pimaric type resin acids can be improved greatly.
4. In the present invention, the crystallized product separated by recrystallization is maleopimaric acid, which can also be taken as a byproduct of the present invention. The obtained maleopimaric acid has high content, and has more active groups than resin acids. The maleopimaric acid has wide application prospects and high economic value in the market, so also can be utilized.
5. The solvent used in the technical method of the present invention is easy to recover and reuse, because it has a low boiling point, and thus has less environmental pollution. This technical process has high operability and economic efficiency, can vary in scale, thus has good industrial application prospects.
6. With the technical method provided in the present invention, by selecting appropriate pine oleoresin, rosin, or resin acids from different kinds of trees as the raw material, pimaric type resin acid products with different composition can be prepared, to meet different demands.

### Brief Description of the Drawings

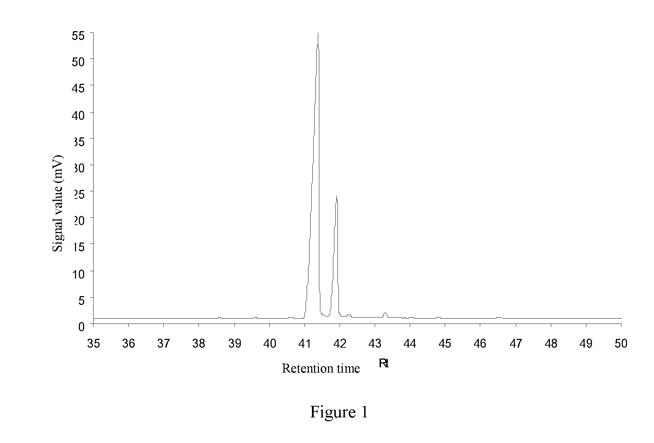

Figure 1 is a GC analytical spectrum of pimaric type resin acid product (treated with methyl ester), which is prepared from *Pinus massoniana* rosin.

The component names and corresponding GC retention times with regard to the component peak numbers in the spectrum are: 1#, 41.38min., pimaric methyl; 2#, 41.92min., sandaracopimaric methyl; 3#, 43.30min., isopimaric methyl; 4#, 44.80min., dehydrogenated abietic methyl; 5#, 46.54min., abietic methyl.

### Detailed Description of the Embodiments

The present invention will be further described in the following examples.

A method for preparing pimaric type resin acids from pine oleoresin or rosin, including gum rosin, tall oil rosin, or wood rosin, preferably *Pinus elliottii* pine oleoresin or *Pinus elliottii* pine rosin, wherein, the pine oleoresin can be transformed into rosin by distilling off turpentine, the pine oleoresin or rosin can be further pre-treated with salinization, acidification, or recrystallization, etc., to remove neutral substances to obtain purified and refined resin acids. Pine oleoresin, rosin, or the refined resin acids obtained are dissolved in glacial acetic acid with a mass of 0.05~30 times; then, maleic anhydride with a mass of 1:0.3∼1.5 times is added into the solution; assist maleation reaction with microwave; add glacial acetic acid with a mass of 0.1∼15 times; cool down and crystallize, then, filter, and wash the precipitate with glacial acetic acid with a mass of 0∼10 times; combine the filtrate, remove the solvent by reduced pressure distillation, and then dissolve the obtained product in 10wt%∼40wt% sodium hydroxide solution, dilute the solution with distilled water or deionized water to a volume (in liters) 10∼500 times of the raw material (pine oleoresin, rosin or purified resin acids obtained from pine oleoresin through pre-treatment). Slowly add hydrochloric acid in drops while agitating, till the pH is in the range of 6∼14. Filter the large amount of produced precipitate and wash it with water, and then dissolve the precipitate in ether with a mass of 0.5∼20 times of the precipitate , then wash with water, till the ether layer becomes neutral, dry the ether layer with drying agent, and then distil off ether at 1 atm; treat the residue by drying and recrystallization to obtain pimaric type resin acids.

### Example 1: Preparation of refined resin acids

Add 1 pbw (part by weight) *Pinus elliottii* pine oleoresin into a three-neck flask with agitator and reflux condenser, add 2.5 pbw petroleum ether with 60°C∼90°C boiling range into the flask, agitate the solution at 40°C till the *Pinus elliottii* pine oleoresin is dissolved completely, remove insoluble solid impurities by filtering before the solution cools down, remove water contained in the pine oleoresin from the filtrate with a separatory funnel, and add the filtrate into a four-neck flask with agitator and reflux condenser, slowly add cyclohexylamine solution in drops while agitating to produce a large quantity of white precipitate of ammonium salt of resin acid, the cyclohexylamine solution is prepared by dissolving cyclohexylamine in quantity equal to the molar quantities of resin acids contained in the pine oleoresin into 0.4 pbw petroleum ether, agitate at 40°C for 1h, and then cool down to room temperature; then, further cool down the solution in ice water bath, and then perform vacuum filtration, wash the precipitate with 0.2 pbw petroleum ether for 3 times, and dry the precipitate in a vacuum drying oven at 40°C; next, grind the precipitate into powder, and add the powder into a three-neck flask that contains 0.7 pbw ether and is equipped with mechanical agitator and ball condenser, agitate to make the white powder suspend in ether, and add 2mol/L hydrochloric acid solution in drops into the flask at room temperature till the white powder disappears completely, and then agitate further for 30min; transfer the mixture into a separatory funnel to remove the water layer, and then wash with distilled water repeatedly till the pH of the water phase is 6, next, remove the water layer and dry the mixture with anhydrous sodium sulfate; distil at 1 atm to remove most of ether in the organic phase, and then transfer the concentrated solution into a watch glass, and dry in vacuum at 40°C to obtain refined resin acids. Take samples, and perform GC analysis and GC-MS analysis.

The samples for GC and GC-MS analysis are pre-treated with methyl ester as follows: dissolve the sample in methanol solution, add phenolphthalein as indicator, add 6wt% methanol solution of tetramethyl ammonium hydroxide in drops, till the sample turns to pink color and the pink color doesn't fade within 30s.

The condition for GC and GC-MS analysis is as follows: two-stage program is employed for heating-up; the initial temperature is 150°C; in the first stage, the heating-up rate is 5°C/min, the final temperature is 220°C, and the holding time is 0min; in the second stage, the heating-up rate is 1°C/min, the final temperature is 270°C, and the holding time is 15min.; the temperature in vaporizing chamber and the temperature of detector are both 260°C; the split ratio is 64:1; the sample size is 0.8µL, the relevant components of pimaric type resin acids are determined by GC-MS analysis, and the mass fraction of pimaric type resin acids is measured with GC normalized method. The result of analysis indicates the total mass fraction of pimaric type resin acids in the *Pinus elliottii* pine resin acids is 19.10wt%.

### Example 2: Preparation of refined resin acids

The *Pinus elliottii* pine oleoresin used in example 1 is replaced with *Pinus massoniana* pine oleoresin, while the rest operations are the same as those in example 1. The result of analysis indicates the total mass fraction of pimaric type resin acids in the *Pinus massoniana* pine resin acids is 7.76wt%.

### Example 3

Add 20.1g refined resin acids prepared according to example 1 into a special microwave reaction bulb, add 8.1g maleic anhydride and 8.2g glacial acetic acid, agitate till the added substances are dissolved completely; arrange the microwave reaction bulb containing the material in a microwave reactor with a reflux condenser, fix the microwave power at 120W, take out the reaction bulb from the microwave reactor after reaction for 28 min; add 47.2g glacial acetic acid into the reaction bulb, and then cool down the solution and let it crystallize, and filter the solution, wash the precipitate with 5g glacial acetic acid, combine the filtrate, distil the solvent at reduced pressure, and then dissolve it in 28wt% sodium hydroxide solution, add water to dilute the solution to 1000mL, slowly add hydrochloric acid in drops while agitating till the pH reaches 7.5, then, filter the precipitate and wash it with water, and then add 17g ether to dissolve the precipitate and wash with water till the pH of the ether layer reaches 8, dry the ether layer with anhydrous sodium sulfate, and then distil off ether at latm, and finally obtain 3.3g pimaric type resin acids after vacuum drying. The yield of pimaric type resin acids is 63.70% of the mass fraction of pimaric type resin acids in the raw material (refined resin acids) (i.e., theoretical yield). The result of GC analysis indicates the mass fraction of pimaric type resin acids in the obtained pimaric type resin acid product is 75.7%.

### Example 4

Add 20.0g refined resin acids prepared according to example 1 into a special microwave reaction bulb, add 8.0g maleic anhydride and 8.0g glacial acetic acid, agitate till the added substances are dissolved completely; arrange the microwave reaction bulb containing the material in a microwave reactor with a reflux condenser, fix the microwave power at 120W, take out the reaction bulb from the microwave reactor after reaction for 25 min; add 10.0g glacial acetic acid into the reaction bulb, cool down the solution and let it crystallize, and then filter the solution, wash the precipitate with 6.0g glacial acetic acid, combine the filtrate, distil the solvent at reduced pressure, and then dissolve it in 20wt% sodium hydroxide solution, add water to dilute the solution to 1000mL, slowly add hydrochloric acid in drops while agitating till the pH reaches 8.5; then, filter the precipitate and wash it with water, and add 15.0g ether to dissolve the precipitate, wash with water till the ether layer becomes neutral, then, dry the ether layer with anhydrous magnesium sulfate, and then distil off ether at 1 atm, finally obtain 2.8g pimaric type resin acid product after vacuum drying. The yield of pimaric type resin acids is 61.8% of the theoretical yield. The result of GC analysis indicates the mass fraction of pimaric type resin acids is 84.30%.

### Example 5

Add 50.0g refined resin acids prepared according to example 2 into a special microwave reaction bulb, add 20.0g maleic anhydride and 20.0g glacial acetic acid, agitate till the added substances are dissolved completely; arrange the microwave reaction bulb containing the material in a microwave reactor with a reflux condenser, fix the microwave power at 120W, take out the reaction bulb from the microwave reactor after reaction for 30min, add 20.0g glacial acetic acid into the reaction bulb, cool down the solution and let it crystallize, and then filter the solution, wash the precipitate with 10.0g glacial acetic acid; combine the filtrate, distil the solvent at reduced pressure, and then dissolve in 35wt% sodium hydroxide solution, add water to dilute the solution to 1000mL, slowly add hydrochloric acid in drops while agitating till the pH reaches 9, then, filter the precipitate and wash it with water, and then add 20.0g ether to dissolve the precipitate, wash with water till the ether layer becomes neutral (pH=7), then, dry the ether layer with anhydrous sodium sulfate, and then distil off ether at 1 atm and finally obtain 2.5g pimaric type resin acid product after vacuum drying,. The yield of pimaric type resin acids is 64.4% of the theoretical yield. The result of GC analysis (shown in Figure 1) indicates the mass fraction of pimaric type resin acids is 93.56%.

### Example 6

Add 20.1g *Pinus elliottii* rosin into a special microwave reaction bulb, add 7.8g maleic anhydride and 8.2g glacial acetic acid, agitate till the added substances are dissolved completely; arrange the microwave reaction bulb containing the material in a microwave reactor with a reflux condenser, fix the microwave power at 120W, take out the reaction bulb from the microwave reactor after reaction for 30 min, add 20.0g glacial acetic acid into the reaction bulb, cool down the solution and let it crystallize, and then filter the solution, wash the precipitate with 5.0g glacial acetic acid, combine the filtrate, distil the solvent at reduced pressure wash the filtrate with 80% ethanol for 2 times, filter the products, dry the filter residue in vacuum and then dissolve it in 28wt% sodium hydroxide solution, add water to dilute the solution to 1000mL, slowly add hydrochloric acid in drops while agitating till the pH reaches 8.2, then, filter the large amount of precipitate and wash it with water, and add 17.0g ether to dissolve the precipitate, wash with water till the ether layer becomes neutral, and then, dry the ether layer with anhydrous sodium sulfate, distil off ether at 1 atm, next, finally obtain 1.8g pimaric type resin acid product after vacuum drying. The yield is 44.2%. The result of GC analysis indicates the mass fraction of pimaric type resin acids is 78.5 %.

### Example 7

Add 1 pbw *Pinus Caribaea* pine rosin into a three-neck flask with agitator and reflux condenser, add 2.5 pbw petroleum ether with 60°C∼90°C boiling range into the flask, agitate the solution at 40°C till the *Pinus Caribaea* pine oleoresin is dissolved completely, remove insoluble solid impurities from the rosin by filtering before the solution cools down, remove water contained in the rosin from the filtrate with a separatory funnel, and add the filtrate into a four-neck flask with agitator and reflux condenser, slowly add cyclohexylamine solution in drops while agitating to produce a large quantity of white precipitate of ammonium salt of resin acid. The cyclohexylamine solution is prepared by dissolving cyclohexylamine in quantity equal to the molar quantities of resin acids contained in the rosin in 0.4 pbw petroleum ether, agitate for 1h at 40°C, and then cool down to room temperature, then further cool down the solution in ice water bath, and perform vacuum filtration, wash the precipitate with 0.2 pbw petroleum ether for 3 times, and dry the precipitate in a vacuum drying oven at 40°C, then grind the precipitate into powder, and add the powder into a three-neck flask containing 0.7 pbw ether and equipped with mechanical agitator and ball condenser, agitate to make the white powder suspend in ether, and add 2mol/L hydrochloric acid solution in drops into the flask at room temperature till the white powder disappears completely, and then agitate further for 30min. transfer the mixture into a separatory funnel to remove the water layer and then wash with distilled water repeatedly till the pH of the water phase reaches 6, remove the water layer and dry the mixture with anhydrous sodium sulfate, distil at 1 atm to remove most of ether in the organic phase, and then transfer the concentrated solution into a watch glass, and dry in vacuum at 40°C to obtain refined resin acids.

Add 20.0g refined resin acids into a special microwave reaction bulb, add 8.0g maleic anhydride and 8.0g butyric acid, agitate till the added substances are dissolved completely; arrange the microwave reaction bulb containing the material in a microwave8 reactor with a reflux condenser, fix the microwave power at 120W, take out the reaction bulb from the microwave reactor after reaction for 25min; add 10.0g butyric acid into the reaction bulb, cool down the solution and let it crystallize, and then filter the solution, wash the precipitate with 6.0g butyric acid, combine the filtrate, distil the solvent at reduced pressure, and then dissolve in 2wt% sodium hydroxide solution, add water to dilute the solution to 1000mL, slowly add 20wt% sulfuric acid in drops while agitating till the pH reaches 8.5, then filter and wash the precipitate with water, add 15.0g ether to dissolve the precipitate, wash with water till the ether layer becomes neutral, then dry the ether layer with anhydrous magnesium sulfate, and then distil off ether at 1 atm, finally obtain pimaric type resin acid product after vacuum drying.

### Example 8

Add 1 pbw *Pinus khasya* pine oleoresin into a three-neck flask with agitator and reflux condenser, add 2.5 pbw petroleum ether with 60°C∼90°C boiling range into the flask, agitate the solution at 40°C till the *Pinus khasya* pine oleoresin is dissolved completely, remove insoluble solid impurities from the pine oleoresin by filtering before the solution cools down, remove water contained in pine oleoresin from the filtrate with a separatory funnel, and add the filtrate into a four-neck flask with agitator and reflux condenser, slowly add cyclohexylamine solution in drops while agitating to produce a large quantity of white precipitate of ammonium salt of resin acid, the cyclohexylamine solution is prepared by dissolving cyclohexylamine in quantity equal to the molar quantities of resin acids contained in the pine oleoresin into 0.4 pbw petroleum ether, agitate for 1h at 40°C, then cool down to room temperature, further cool down the solution in ice water bath, then perform vacuum filtration, wash the precipitate with 0.2 pbw petroleum ether for 3 times, and dry the precipitate in a vacuum drying oven at 40°C, grind the precipitate into powder, and add the powder into a three-neck flask that contains 0.7 pbw ether and is equipped with mechanical agitator and ball condenser, agitate to make the white powder suspend in ether, and add 2mol/L hydrochloric acid solution in drops into the flask at room temperature till the white powder disappears completely, and then agitate further for 30min, transfer the mixture into a separatory funnel to remove the water layer, then wash with distilled water repeatedly till the pH of the water phase reaches 6, remove the water layer and dry the mixture with anhydrous sodium sulfate, distil at 1 atm to remove most of ether in the organic phase, then transfer the concentrated solution into a watch glass, and dry in vacuum at 40°C to obtain refined resin acids.

Add 20.0g refined resin acids into a special microwave reaction bulb, add 8.0g maleic anhydride and 8.0g propionic acid, agitate till the added substances are dissolved completely, arrange the microwave reaction bulb containing the material in a microwave reactor with a reflux condenser, fix the microwave power at 120W, take out the reaction bulb from the microwave reactor after reaction for 25 min; add 10.0g propionic acid into the reaction bulb, cool down the solution and let it crystallize, then filter and wash the precipitate with 6.0g propionic acid, combine the filtrate, distil the solvent at reduced pressure and then dissolve in 40wt% sodium hydroxide solution, add water to dilute the solution to 1000mL, slowly add 1wt% hydrochloric acid in drops while agitating till the pH reaches 8.5, then, filter the precipitate and wash it with water, and add 15.0g ether to dissolve the precipitate, and wash with water till the ether layer becomes neutral, then dry the ether layer with anhydrous magnesium sulfate, then distil off ether at 1 atm, and finally obtain pimaric type resin acid product after vacuum drying.

## Claims

1. A process for preparing pimaric type resin acids, wherein the process includes the following steps:
step 1: add refined resin acids, pine resin or rosin, and maleic anhydride at the mass ration of 1:0.3∼1.5 into a reaction bulb, dissolve them in C₁∼C₁₀ lower fatty acid solvent, the mass ratio between C₁∼C₁₀ lower fatty acid solvent and refined resin acids is 0.05∼30:1, and then perform additive reaction under microwave-assisted heating or direct heating, after the reaction, let the solution cool down and crystallize, then filter and wash;
step 2: combine the filtrate collected in step 1, then perform a reduced pressure distillation to remove the solvent to obtain the crude product of pimaric type resin acids, dissolve the crude product of pimaric type resin acids in sodium hydroxide solution to produce saline solution of pimaric type resin acids, then, adjust the pH to 6∼14 with mineral acid or organic acid while agitating, purify the obtained precipitate to obtain the final product of pimaric type resin acids, wherein the final product of pimaric type resin acids comprises a mixture of pimaric type resin acids.

2. The process for preparing pimaric type resin acid according to claim 1, wherein, the condition of microwave-assisted heating is as follows: the microwave power is 100W∼50kW, and the reaction time under microwave heating is 5min∼300min.

3. The process for preparing pimaric type resin acids according to claim 1, wherein, the refined resin acids are products obtained by removing neutrals from pine oleoresin or rosin by recrystallization in organic solvent, treating with sodium salt, treating with ammonium salt, or washing with organic solvent.

4. The process for preparing pimaric type resin acids according to claim 2, wherein, the pine oleoresin is any one of *Pinus massoniana* pine oleoresin, *Pinus elliottii* pine oleoresin, *Pinus Caribaea* pine oleoresin, and *Pinus khasya* pine oleoresin; the rosin is any one of gum rosin, tall oil rosin, and wood rosin which are produced from *Pinus massoniana* pine, *Pinus Caribaea* pine, *Pinus khasya* pine, or *Pinus elliottii* pine.

5. The process for preparing pimaric type resin acids according to claim 1, wherein, the C₁∼C₁₀ lower fatty acid solvent is any one of glacial acetic acid, propionic acid, and butyric acid.

6. The process for preparing pimaric type resin acids according to claim 1, wherein, the mass fraction of sodium hydroxide solution is 0.5%∼40%.

7. The process for preparing pimaric type resin acids according to claim 1, wherein, the mineral acid in step 2 is hydrochloric acid or sulfuric acid, the organic acid is formic acid or acetic acid, and the mass fraction of the acid is 0.5%∼100%.

8. The process for preparing pimaric type resin acids according to claim 1, wherein, the purification treatment is to dissolve the precipitate with ether with a mass of 0.5∼20 times of the precipitate, wash with water till the ether layer become neutral, and then dry with anhydrous magnesium sulfate, distill off ether at 1 atm, and finally dry in vacuum to obtain the product of pimaric type resin acids.

9. The process for preparing pimaric type resin acids according to claim 1, wherein the refined resin acids are obtained as follows:
providing oleoresin or rosin, recrystallizing the oleoresin or rosin in an organic solvent,
treating the recrystallized oleoresin or rosin with sodium salt or ammonium salt, or washing the recrystallized oleoresin or rosin with organic solvent to obtained refined resin acids.

10. The process for preparing pimaric type resin acids according to claim 1, wherein the final product of pimaric type resin acids contains at least 93.5% pimaric type resin acids, and a yield of at least 64.4%.

## Patentansprüche

1. Verfahren zur Herstellung von Harzsäuren des Pimarsäuretyps, wobei das Verfahren die folgenden Schritte umfasst:
Schritt 1: Einfüllen raffinierter Harzsäuren, Kiefernharz oder Kolophonium sowie Maleinsäureanhydrid in einem Massenverhältnis von 1:0,3-1,5 in einen Reaktionskolben, Auflösen derselben in einem C₁-C₁₀-Niederfettsäure-Lösungsmittel, wobei das Massenverhältnis von C₁-C₁₀-Niederfettsäure-Lösungsmittel zu raffinierten Harzsäuren 0,05-30:1 beträgt, und dann Durchführen einer Additionsreaktion unter mikrowellengestütztem Erhitzen oder direktem Erhitzen, nach der Reaktion die Lösung abkühlen und kristallisieren lassen, dann Filtrieren und Waschen;
Schritt 2: Vereinigen des in Schritt 1 aufgefangenen Filtrats, dann Durchführen einer Destillation unter reduziertem Druck zur Entfernung des Lösungsmittels, wobei man das Rohprodukt von Harzsäuren des Pimarsäuretyps erhält, Auflösen des Rohprodukts von Harzsäuren des Pimarsäuretyps in Natronlauge unter Bildung einer Lösung von Harzsäuren des Pimarsäuretyps in einer salzigen Lösung, dann Einstellen des pH-Werts auf 6-14 mit Mineralsäure oder organischer Säure unter Rühren, Reinigen des erhaltenen Niederschlags, wobei man das Endprodukt von Harzsäuren des Pimarsäuretyps erhält, wobei das Endprodukt von Harzsäuren des Pimarsäuretyps ein Gemisch von Harzsäuren des Pimarsäuretyps umfasst.

2. Verfahren zur Herstellung von Harzsäuren des Pimarsäuretyps gemäß Anspruch 1, wobei die Bedingungen des mikrowellengestützten Erhitzens wie folgt sind: die Mikrowellenleistung beträgt 100 W-50 kW, und die Reaktionszeit unter Mikrowellenheizung beträgt 5 min bis 300 min.

3. Verfahren zur Herstellung von Harzsäuren des Pimarsäuretyps gemäß Anspruch 1, wobei die raffinierten Harzsäuren Produkte sind, die durch Entfernen der neutralen Substanzen aus Kiefernfettharz oder Kolophonium durch Umkristallisieren aus einem organischen Lösungsmittel, Behandeln mit Natriumsalz, Behandeln mit Ammoniumsalz oder Waschen mit organischem Lösungsmittel erhalten werden.

4. Verfahren zur Herstellung von Harzsäuren des Pimarsäuretyps gemäß Anspruch 2, wobei das Kiefernfettharz aus *Pinus-massionana*-Kiefernfettharz, *Pinus-elliottii*-Kiefernfettharz, *Pinus-caribaea-*Kiefernfettharz und *Pinus-khasya*-Kiefernfettharz ausgewählt ist, das Kolophonium aus Balsamharz, Tallharz und Wurzelharz, die aus *Pinus-massionana-*Kiefer, *Pinus-caribaea*-Kiefer, *Pinus-khasya-*Kiefer oder *Pinus-elliottii*-Kiefer hergestellt sind, ausgewählt ist.

5. Verfahren zur Herstellung von Harzsäuren des Pimarsäuretyps gemäß Anspruch 1, wobei das C₁-C₁₀-Niederfettsäure-Lösungsmittel aus Eisessig, Propionsäure und Buttersäure ausgewählt ist.

6. Verfahren zur Herstellung von Harzsäuren des Pimarsäuretyps gemäß Anspruch 1, wobei der Massenanteil der Natronlauge 0,5%-40% beträgt.

7. Verfahren zur Herstellung von Harzsäuren des Pimarsäuretyps gemäß Anspruch 1, wobei es sich bei der Mineralsäure in Schritt 2 um Salzsäure oder Schwefelsäure und bei der organischen Säure um Ameisensäure oder Essigsäure handelt und der Massenanteil der Säure 0,5%-100% beträgt.

8. Verfahren zur Herstellung von Harzsäuren des Pimarsäuretyps gemäß Anspruch 1, wobei die Reinigungsbehandlung darin besteht, den Niederschlag mit Ether mit einer Masse vom 0,5-20-fachen des Niederschlags aufzulösen, mit Wasser zu waschen, bis die Etherschicht neutral wird, und dann mit wasserfreiem Magnesiumsulfat zu trocknen, Ether bei 1 atm abzudestillieren und schließlich im Vakuum zu trocknen, wobei man das Produkt der Harzsäuren des Pimarsäuretyps erhält.

9. Verfahren zur Herstellung von Harzsäuren des Pimarsäuretyps gemäß Anspruch 1, wobei die raffinierten Harzsäuren wie folgt erhalten werden:
Bereitstellen von Fettharz oder Kolophonium, Umkristallisieren des Fettharzes oder Kolophoniums aus einem organischen Lösungsmittel;
Behandeln des umkristallisierten Fettharzes oder Kolophoniums mit Natriumsalz oder Ammoniumsalz oder Waschen des umkristallisierten Fettharzes oder Kolophoniums mit organischem Lösungsmittel, wobei man raffinierte Harzsäuren erhält.

10. Verfahren zur Herstellung von Harzsäuren des Pimarsäuretyps gemäß Anspruch 1, wobei das Endprodukt von Harzsäuren des Pimarsäuretyps wenigstens 93,5% Harzsäuren des Pimarsäuretyps und eine Ausbeute von wenigstens 64,4% enthält.

## Revendications

1. Procédé pour la préparation d'acides résiniques de type pimarique, dans lequel le procédé comprend les étapes suivantes :
étape 1 : ajouter des acides résiniques raffinés, de la résine de pin ou de la colophane, et de l'anhydride maléique selon un rapport de masse de 1 : 0,3 à 1,5 dans un ballon à réaction, dissoudre ceux-ci dans un solvant d'acide gras inférieur en C₁ à C₁₀, le rapport de masse entre le solvant d'acide gras inférieur en C₁ à C₁₀ et les acides résiniques raffinés étant 0,05 à 30 : 1, et ensuite réaliser une réaction d'addition avec chauffage assisté par micro-ondes ou chauffage direct, et après la réaction, laisser la solution refroidir et se cristalliser, puis filtrer et laver,
étape 2 : combiner le filtrat recueilli dans l'étape 1, ensuite réaliser une distillation à pression réduite pour enlever le solvant pour obtenir le produit brut des acides résiniques de type pimarique, dissoudre le produit brut des acides résiniques de type pimarique dans une solution d'hydroxyde de sodium pour produire une solution saline des acides résiniques de type pimarique, ensuite ajuster le pH à 6 à 14 avec un acide minéral ou un acide organique en agitant, purifier le précipité obtenu pour obtenir le produit final des acides résiniques de type pimarique, dans lequel le produit final des acides résiniques de type pimarique comprend un mélange d'acides résiniques de type pimarique.

2. Procédé pour la préparation d'acides résiniques de type pimarique selon la revendication 1, dans lequel les conditions du chauffage assisté par micro-ondes sont comme suit : la puissance micro-ondes est 100 W à 50 kW, et le temps de réaction sous chauffage micro-ondes est 5 min à 300 min.

3. Procédé pour la préparation d'acides résiniques de type pimarique selon la revendication 1, dans lequel les acides résiniques raffinés sont des produits obtenus par l'élimination des substances neutres d'oléorésine de pin ou de colophane par recristallisation dans un solvant organique, traitement avec un sel de sodium, traitement avec un sel d'ammonium, ou lavage avec un solvant organique.

4. Procédé pour la préparation d'acides résiniques de type pimarique selon la revendication 2, dans lequel l'oléorésine de pin est choisie parmi l'oléorésine de pin de *Pinus massionana,* l'oléorésine de pin de *Pinus elliottii,* l'oléorésine de pin de *Pinus caribaea* et l'oléorésine de pin de *Pinus khasya,* la colophane est choisie parmi la colophane de gemme, la colophane de tallol et la colophane de bois, qui sont produites à partir du pin *Pinus massionana,* du pin *Pinus caribaea,* du pin *Pinus khasya* et du pin *Pinus elliottii*.

5. Procédé pour la préparation d'acides résiniques de type pimarique selon la revendication 1, dans lequel le solvant d'acide gras inférieur en C₁ à C₁₀ est choisi parmi l'acide acétique glacial, l'acide propionique, et l'acide butyrique.

6. Procédé pour la préparation d'acides résiniques de type pimarique selon la revendication 1, dans lequel la fraction massique de la solution d'hydroxyde de sodium est 0,5 % à 40 %.

7. Procédé pour la préparation d'acides résiniques de type pimarique selon la revendication 1, dans lequel ledit acide minéral dans l'étape 2 est l'acide hydrochlorique ou l'acide sulfurique, ledit acide organique est l'acide formique ou l'acide acétique, et la fraction massique de l'acide est 0,5 % à 100 %.

8. Procédé pour la préparation d'acides résiniques de type pimarique selon la revendication 1, dans lequel le traitement de purification consiste à dissoudre le précipité avec de l'éther ayant une masse de 0,5 à 20 fois celle du précipité, laver à l'eau jusqu'à ce que la phase éthérée devienne neutre, et ensuite sécher avec du sulfate de magnésium anhydre, enlever l'éther par distillation à 1 atm, et finalement sécher sous vide pour obtenir le produit des acides résiniques de type pimarique.

9. Procédé pour la préparation d'acides résiniques de type pimarique selon la revendication 1, dans lequel les acides résiniques raffinés sont obtenus comme suit :
procurer l'oléorésine ou la colophane, recristalliser l'oléorésine ou la colophane dans un solvant organique,
traiter l'oléorésine ou la colophane recristallisée avec un sel de sodium ou un sel d'ammonium, ou laver l'oléorésine ou la colophane recristallisée avec un solvant organique pour obtenir les acides résiniques raffinés.

10. Procédé pour la préparation d'acides résiniques de type pimarique selon la revendication 1, dans lequel le produit final des acides résiniques de type pimarique contient au moins 93,5 % d'acides résiniques de type pimarique, et un rendement d'au moins 64,4 %.
